# DEMANDE DE BREVET EUROPEEN

(11) **EP 4 553 145 A1**
(43) Date de publication de la demande: **14.05.2025**
(21) Numéro de dépôt: 24209385.4
(22) Date de dépôt: 29.10.2024
(51) Int. Cl.: C12M 1/42, C12M 1/22, C12M 1/00

(54) **SYSTÈME DE STIMULATION LUMINEUSE D'UN ÉCHANTILLON BIOLOGIQUE**

(30) Priorité: 09.11.2023 FR 2312260
(71) Demandeur: Commissariat à l'Energie Atomique et aux Energies Alternatives, 75015 Paris (FR)
(72) Inventeur: BLEUET, Pierre, 38054 GRENOBLE CEDEX 09 (FR)
(74) Mandataire: INNOV-GROUP

(57) **Abrégé**

L'invention concerne un système de stimulation lumineuse d'un échantillon biologique (ECH), comprenant :
- Une source lumineuse (2) apte à émettre un faisceau lumineux, dit faisceau lumineux d'entrée (F1),
- Une enceinte (3) fermée destinée à recevoir au moins un contenant (1) dans lequel est placé l'échantillon biologique,
- Le système comportant un dispositif optique (5),
- Le système comportant un guide optique (4) connecté d'un côté sur ladite source lumineuse (2) pour recevoir ledit faisceau lumineux d'entrée (F1) et de l'autre côté sur ledit dispositif optique (5), le guide optique (4) étant agencé pour guider le faisceau lumineux d'entrée émis par la source lumineuse jusqu'au dispositif optique (5) et émettre un faisceau lumineux, dit faisceau lumineux de sortie (F2),
- Ledit dispositif optique étant configuré pour générer un faisceau lumineux parallèle de stimulation (F4) avec une section constante déterminée, à partir dudit faisceau lumineux de sortie (F2).

## Description

### Domaine technique de l'invention

La présente invention se rapporte à un système de stimulation lumineuse d'un échantillon biologique.

### Etat de la technique

La photobiomodulation ou la photothérapie sont des techniques en plein essor qui visent à guérir ou ralentir une pathologie à l'aide de la lumière. La photobiomodulation couvre un large spectre d'application, dans le domaine du traitement des maladies neurodégénératives, de la fibromyalgie ou de la douleur.

Pour évaluer l'efficacité de la photobiomodulation sur un échantillon biologique tel que des cellules, il est connu de venir positionner des boîtes de Petri contenant les cellules sur un panneau lumineux, ce plateau étant lui-même placé dans une enceinte fermée pour rester dans un environnement régulé en température (par exemple 37°C) et en humidité (80-90% d'humidité). Le panneau lumineux génère ainsi un flux lumineux à travers chaque boîte de Petri disposé sur le panneau.

Cependant, à l'usage, cette configuration génère quelques inconvénients :
- Lors de tests longs de plusieurs heures la carte électronique du panneau lumineux a tendance à chauffer, ce qui augmente la température à l'intérieur de l'enceinte, pouvant entraîner une dégradation des cellules ; cela oblige à positionner le panneau d'illumination sur un panneau refroidissant (par exemple avec une circulation d'eau régulée en température) pour dissiper la chaleur générée par la carte électronique. Le réglage de la consigne en température à l'intérieur de l'enceinte et la gestion de la circulation d'eau du panneau refroidissant deviennent difficiles et le point de fonctionnement instable. La température de consigne du panneau refroidissant étant basse pour évacuer la chaleur efficacement et le taux d'humidité dans l'enceinte étant réglé à 80% environ, on dépasse le point de rosée et la condensation devient alors un problème (oxydation, risque électrique, taux d'humidité).
- Le panneau d'illumination entier émet de la lumière, ce qui n'est pas nécessaire car seules les boites de Petri doivent être illuminées ; cela conduit à des réflexions lumineuses qui peuvent influer sur l'étude biologique et à une surconsommation venant influer sur la température à l'intérieur de l'enceinte.
- Les diodes électroluminescentes du panneau d'illumination sont connues pour vieillir de manière accélérée avec la température. Par exemple, on peut avoir une perte de rendement lumineux de 1 0% par an en utilisation intensive, nécessitant ainsi un remplacement fréquent du panneau d'illumination pour conserver des performances acceptables.

Des solutions de stimulation sont décrites dans les demandes de brevet CN112899157, JP2006174764.

Le but de l'invention est de proposer un système employé pour la stimulation lumineuse d'un échantillon biologique, ce système ayant une configuration permettant de pallier les inconvénients de l'état de la technique.

### Exposé de l'invention

Ce but est atteint par un système de stimulation lumineuse d'un échantillon biologique, comprenant :
- Une source lumineuse apte à émettre un faisceau lumineux, dit faisceau lumineux d'entrée,
- Une enceinte fermée destinée à recevoir au moins un contenant dans lequel est placé l'échantillon biologique,
- La source lumineuse étant placée à l'extérieur de l'enceinte,
- Le système comportant un dispositif optique placé à l'intérieur de l'enceinte,
- Le système comportant un guide optique connecté d'un côté sur ladite source lumineuse pour recevoir ledit faisceau lumineux d'entrée et de l'autre côté sur ledit dispositif optique, le guide optique étant agencé pour guider le faisceau lumineux d'entrée émis par la source lumineuse jusqu'au dispositif optique et émettre un faisceau lumineux, dit faisceau lumineux de sortie, à destination du dispositif optique,
- Ledit dispositif optique étant configuré pour générer un faisceau lumineux parallèle de stimulation de l'échantillon biologique avec une section constante déterminée à partir dudit faisceau lumineux de sortie.

Selon une particularité, le dispositif optique comporte un ensemble optique configuré pour générer un faisceau lumineux divergent à partir du faisceau lumineux de sortie et un miroir parabolique hors axe positionné par rapport audit ensemble optique pour réfléchir ledit faisceau lumineux divergent et former ledit faisceau lumineux parallèle de stimulation.

Selon une autre particularité, le système comporte des moyens de réglage de la position dudit ensemble optique par rapport à celle du miroir parabolique hors axe.

Selon une autre particularité, le système comporte des moyens de réglage de la position du miroir parabolique hors axe par rapport à celle dudit ensemble optique.

Selon une autre particularité, le système comporte des moyens de réglage de l'orientation dudit ensemble optique par rapport au miroir parabolique hors axe.

Selon une autre particularité, le système comporte des moyens de réglage de l'orientation du miroir parabolique hors axe par rapport audit ensemble optique.

Selon une autre particularité, la source lumineuse est une source laser régulée.

Selon une autre particularité, le système comporte un masque agencé en sortie du dispositif optique pour mettre en forme la section du faisceau lumineux parallèle de stimulation.

Selon une autre particularité, le guide d'onde comporte un séparateur, agencé pour séparer ledit faisceau lumineux d'entrée en ledit faisceau lumineux de sortie et un deuxième faisceau lumineux de sortie, le système comportant un deuxième dispositif optique de mise en forme du deuxième faisceau lumineux de sortie, configuré pour générer un deuxième faisceau lumineux de stimulation.

Selon une autre particularité, l'enceinte fermée est un incubateur.

L'invention concerne également un procédé de stimulation lumineuse d'un échantillon biologique, mis en oeuvre à l'aide du système tel que défini ci-dessus, le procédé comportant les étapes suivantes :
- Positionner dans ladite enceinte fermée un contenant dans lequel est placé ledit échantillon biologique à stimuler, ledit contenant comportant une fenêtre d'illumination ayant une section d'illumination par laquelle peut pénétrer un faisceau lumineux, ce faisceau lumineux étant un faisceau lumineux parallèle de stimulation de l'échantillon biologique,
- Configurer le dispositif optique pour obtenir le faisceau lumineux parallèle de stimulation avec une section constante de surface supérieure ou égale à la section d'illumination de la fenêtre d'illumination du contenant,
- Activer ladite source lumineuse pour émettre le faisceau lumineux d'entrée à l'intérieur du guide optique et obtenir le faisceau lumineux de sortie à appliquer au dispositif optique pour la mise en forme de ce faisceau.

Selon une particularité, le contenant est une boîte de Petri.

### Brève description des figures

D'autres caractéristiques et avantages vont apparaître dans la description détaillée qui suit faite en regard des dessins annexés dans lesquels :

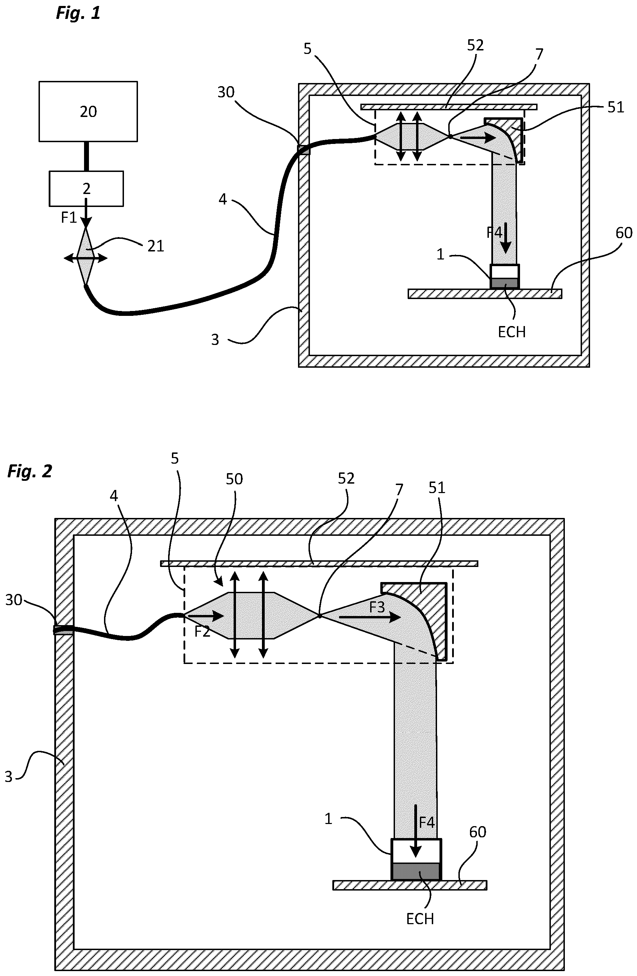

- La figure 1 représente, de manière schématique, le système de stimulation lumineuse d'un échantillon biologique, conforme à l'invention ;
- La figure 2 montre de manière schématique l'architecture de la partie finale du système de stimulation de l'invention ;
- La figure 3 montre le principe de réalisation du faisceau lumineux de stimulation venant illuminer le contenant l'échantillon biologique ;
- La figure 4 montre par une vue en perspective un exemple de réalisation du dispositif optique employé dans le système de l'invention ;
- La figure 5 montre, de manière schématique, une réalisation avantageuse du système de stimulation lumineuse d'un échantillon biologique conforme à l'invention ;
- La figure 6 montre, de manière schématique, une variante de réalisation du dispositif optique employé dans le système de l'invention ;

### Description détaillée d'au moins un mode de réalisation

L'invention s'applique à la stimulation lumineuse d'un échantillon biologique ECH. Par échantillon biologique ECH, on entend par exemple des cellules d'un être vivant, mais également toute autre substance animale ou végétale pour laquelle une stimulation lumineuse pourrait avoir un effet.

L'invention s'applique plus particulièrement à la culture cellulaire, plus particulièrement à l'influence de la lumière sur la culture de cellules et à la surveillance de l'effet de la lumière sur la culture de cellules. Par les termes « lumière » ou « faisceau lumineux » employé ci-après, on entend tout rayonnement électromagnétique dont la longueur d'onde va de l'ultraviolet à l'infrarouge lointain en passant par la lumière visible.

### Contenant

### Figure 3

L'échantillon biologique ECH est placé dans un contenant 1. Ce contenant 1 comporte une paroi latérale et une paroi de fond et peut être ouvert sur le dessus ou fermé par une paroi transparente à une ou plusieurs longueurs d'ondes du faisceau lumineux émis par la source lumineuse du système. La zone traversée par le faisceau lumineux définit une fenêtre d'illumination 10 de l'échantillon biologique ECH. Cette fenêtre d'illumination 10 présente une section transversale délimitant ladite zone traversée par le faisceau lumineux. Cette section transversale est avantageusement circulaire. On verra cependant ci-après que cette section pourrait être différente et quelconque, moyennant une légère adaptation du système.

Le contenant 1 est par exemple réalisé sous la forme d'une boîte de Petri.

Le système de stimulation lumineuse de l'invention comporte principalement :
- Une source lumineuse 2 ;
- Une enceinte 3 ;
- Un guide optique 4 ;
- Un dispositif optique 5 ;

### Source lumineuse

### Figure 1

### Figure 2

Le système comporte au moins une source lumineuse 2. Cette source lumineuse 2 est alimentée pour générer un faisceau lumineux, appelé ici faisceau lumineux d'entrée F1. La source lumineuse 2 peut être constituée d'une ou plusieurs diodes électroluminescentes ou avantageusement d'au moins une diode Laser.

La source lumineuse 2 est avantageusement régulée en intensité.

Dans le domaine de la photobiomodulation, la source lumineuse 2 est choisie pour émettre à une longueur d'onde comprise entre 600nm et 1000nm.

Dans un exemple de réalisation, on utilise un boitier de contrôle 20 qui permet d'alimenter en courant une diode laser de longueur d'onde 670 nm, émettant au maximum 1.3 W. Le courant délivré par le boîtier d'alimentation est de l'ordre de 1 ampère. Ce même boitier de contrôle 20 permet de réguler en température la diode Laser qui est montée sur une mécanique dédiée.

Il faut noter que la diode Laser émet le faisceau lumineux d'entrée F1 qui est très divergent et qu'il convient alors de refocaliser le faisceau avec un jeu de lentilles 21 pour guider la lumière efficacement vers un guide optique (voir ci-après), l'ouverture numérique du jeu de lentilles étant inférieure à l'ouverture numérique du guide optique 4 pour un couplage optimal.

Selon l'invention, la source lumineuse 2 est positionnée à l'extérieur de l'enceinte 3 fermée 3.

### Enceinte fermée

### Figure 1

### Figure 2

Le système comporte une enceinte 3 fermée dans lequel est positionné ledit contenant 1 recevant l'échantillon biologique ECH.

Cette enceinte 3 est fermée de manière étanche. Dans le cadre de la culture cellulaire, l'espace interne de l'enceinte 3 est avantageusement régulé en température et en humidité pour obtenir des conditions optimales à la croissance cellulaire. A titre d'exemple, la température peut être régulée à 37°C et l'humidité à 80% environ. Un boîtier de contrôle spécifique peut être configuré pour gérer la température et le taux d'humidité à l'intérieur de l'enceinte 3.

L'enceinte 3 est par exemple un incubateur. Cet incubateur peut comporter au moins une traversée 30 étanche formée à travers l'une de ses parois, cette traversée étant adaptée pour y insérer un guide optique 4.

De manière avantageuse, l'enceinte est fermée de manière à disposer de parois complètement opaques à la lumière externe et à former un volume interne le plus sombre possible.

Avantageusement, la surface interne de l'enceinte peut être recouverte d'un matériau qui absorbe les longueurs d'ondes de la photobiomodulation, en vue d'éviter les réflexions secondaires qui perturbent la stimulation.

### Guide optique

### Figure 1

### Figure 2

Pour acheminer le faisceau lumineux d'entrée F1 émis par la source lumineuse 2 vers l'intérieur de l'enceinte 3, le système comporte un guide optique 4, par exemple une fibre optique.

Comme indiqué ci-dessus, dans le cas d'une source lumineuse de type diode Laser ou diode électroluminescente qui émet un faisceau très divergent, il peut s'avérer nécessaire d'utiliser un jeu de lentilles en entrée du guide optique 4 et de faire en sorte que l'ouverture numérique de la lentille soit inférieure à l'ouverture numérique du guide optique pour un couplage optimal.

Le guide optique 4 pénètre à travers l'enceinte 3, via la traversée 30 dédiée. Le faisceau lumineux d'entrée F1 émis par la source lumineuse 2, située à l'extérieur de l'enceinte, est donc acheminé à l'intérieur de l'enceinte via le guide optique 4.

Il faut noter que le guide optique 4 présente des caractéristiques de souplesse lui permettant d'être légèrement courbé et manipulé, facilitant la mise en place globale du système.

### Dispositif optique

### Figure 1

### Figure 2

### Figure 3

### Figure 4

Le dispositif optique 5 est positionné à l'intérieur de l'enceinte 3, en sortie du guide optique 4. Sa fonction est de récupérer le faisceau lumineux obtenu en sortie du guide optique et dit faisceau lumineux de sortie F2, afin de mettre en forme ce faisceau.

Le dispositif optique 5 est configuré pour mettre en forme le faisceau lumineux de sortie F2 en vue d'obtenir le faisceau lumineux parallèle de stimulation F4 de l'échantillon biologique ECH souhaité.

Selon l'invention, l'objectif est en effet d'obtenir un faisceau lumineux parallèle de stimulation F4 disposant d'une section constante, avantageusement circulaire, sur toute sa longueur, entre le dispositif optique 5 et le contenant 1 recevant l'échantillon biologique ECH.

La section circulaire du faisceau F4 obtenue est avantageusement supérieure ou égale à la section circulaire de la fenêtre d'illumination 10 du contenant 1.

Le dispositif optique 5 comporte un ensemble optique 50 configuré pour générer un faisceau lumineux divergent F3 à partir du faisceau lumineux de sortie F2 et un miroir parabolique hors axe 51 destiné à recevoir ledit faisceau lumineux divergent F3 pour le réfléchir et former ledit faisceau lumineux parallèle de stimulation F4.

L'ensemble optique 50 peut être composé de deux lentilles convergentes positionnées en série de manière adaptée et non accolées. En sortie de la deuxième lentille, le faisceau obtenu converge d'abord vers un point source qui forme ainsi une source secondaire 7 de lumière. L'ensemble optique 50 est conçu de manière à ce que cette source secondaire 7 soit positionnée à la distance focale du miroir parabolique hors-axe 51, ce qui permet de s'assurer que le faisceau F4 en sortie du miroir 51 soit parallèle et non divergent. Cette source secondaire 7 est divergente en direction du miroir parabolique hors axe 51. L'ensemble optique 50 permet donc de créer cette source secondaire 7 divergente intermédiaire à partir du faisceau lumineux de sortie F2.

Pour rappel, un miroir parabolique hors axe 51 est un dispositif optique connu, doté d'une surface réfléchissante courbe, ayant un profil parabolique. Il a la capacité de renvoyer un flux lumineux parallèle à partir d'une source lumineuse divergente.

En sortie du guide optique 4, l'ensemble optique 50 et sa position doivent être choisies pour que la divergence du faisceau lumineux F3 généré soit suffisante pour illuminer une large partie du miroir parabolique hors axe 51 et s'assurer d'obtenir un faisceau lumineux réfléchi F4 utilisé pour la stimulation qui soit de section constante et suffisante par rapport à la section de la fenêtre d'illumination 10 du contenant 1. Autrement dit, l'ensemble optique 50 doit être adapté pour focaliser le faisceau lumineux et former la source secondaire 7 correspondant à la focale du miroir parabolique hors axe.

A titre d'exemple, un miroir parabolique hors axe de 3 pouces (76.2 mm) permettra d'illuminer une boite de Petri conventionnelle.

Le faisceau lumineux parallèle de stimulation F4 généré permet d'avoir une homogénéité d'illumination sur toute la fenêtre d'illumination 10 du contenant 1, et de pouvoir venir éclairer uniquement ledit contenant 1, à condition bien entendu que le diamètre du miroir parabolique hors axe 51 soit choisi supérieur ou égal au diamètre de la fenêtre d'illumination 10 (par exemple le diamètre de la boîte de Petri).

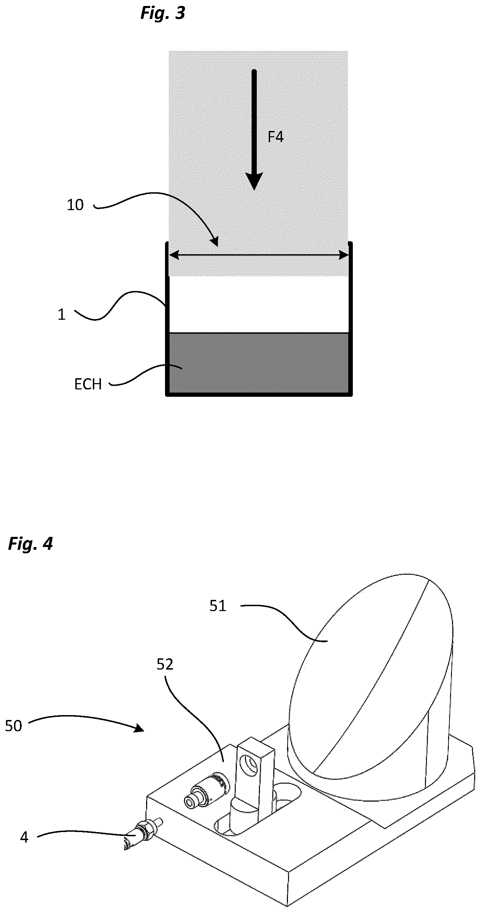

De manière avantageuse, comme représenté sur la figure 4, le dispositif optique 5 peut être réalisé en un seul bloc optique comportant un support 52 sur lequel est agencé l'ensemble optique 50 et le miroir parabolique hors axe 51. L'ensemble optique 50 peut notamment être monté en coulissement dans ledit support 52 pour régler sa position par rapport au miroir parabolique hors axe 51. De manière similaire, il serait également possible de venir régler la position du miroir parabolique hors axe.

Par ailleurs, on pourrait prévoir des moyens de réglage de l'orientation de l'ensemble optique 50 et/ou du miroir parabolique hors axe 51 afin de pouvoir les orienter l'un par rapport à l'autre.

Si la section de la fenêtre d'illumination 10 du contenant n'est pas circulaire ou si le faisceau lumineux parallèle de stimulation F4 présente une section trop étendue par rapport à celle de la fenêtre d'illumination 10 du contenant, le système peut comporter un masque pour venir modifier la section du faisceau lumineux parallèle de stimulation F4, afin que celle-ci épouse au plus près la forme de la fenêtre d'illumination 10 du contenant. Dans ce cas, on comprend que l'invention ne s'applique pas forcément au cas où la fenêtre d'illumination 10 du contenant présente une section circulaire.

### Agencement du système

### Figure 1

### Figure 2

### Figure 3

### Figure 4

Comme indiqué ci-dessus, la source lumineuse 3 est positionnée à l'extérieur de l'enceinte 3.

Le guide optique 4 est connecté sur la source lumineuse pour acheminer le faisceau lumineux d'entrée émis par la source vers l'intérieur de l'enceinte.

De l'autre côté, le guide optique 4 est connecté sur l'ensemble optique 50 du dispositif optique 5. Le faisceau lumineux F2 obtenu en sortie du guide optique 4 est envoyé sur l'ensemble optique 50.

Des connectiques optiques sont prévues pour connecter chaque extrémité du guide optique 4.

L'ensemble optique 50 permet de générer la source divergente créant le faisceau lumineux divergent F3 en direction du miroir parabolique hors axe 51.

Le miroir parabolique hors axe 51 reçoit le faisceau lumineux divergent F3 et le réfléchit pour obtenir le faisceau lumineux parallèle de stimulation F4 recherché, à destination du contenant 1.

Le contenant 1 est par exemple posé sur un premier support 60 plan présent dans l'enceinte 3 fermée, sa fenêtre d'illumination 10 étant alors parallèle à ce premier support.

Le dispositif optique peut être fixé par l'intermédiaire de son support 52 sur une plaque montée parallèlement au support 60 supportant le contenant 1, de sorte que le miroir parabolique hors axe 51 émette le faisceau lumineux parallèle de stimulation F4 suivant une direction perpendiculaire à la fenêtre d'illumination 10. Cette plaque pourra venir supporter plusieurs dispositifs optiques 5 en parallèle, afin de pouvoir traiter plusieurs échantillons en parallèle.

Comme le faisceau lumineux est acheminé vers le dispositif optique 5 par le guide optique 4, il n'est pas nécessaire d'orienter spécifiquement la source lumineuse 2 par rapport au dispositif optique 5, ce qui facilite la mise en place du système.

### Variantes de réalisation

### Figure 5

### Figure 6

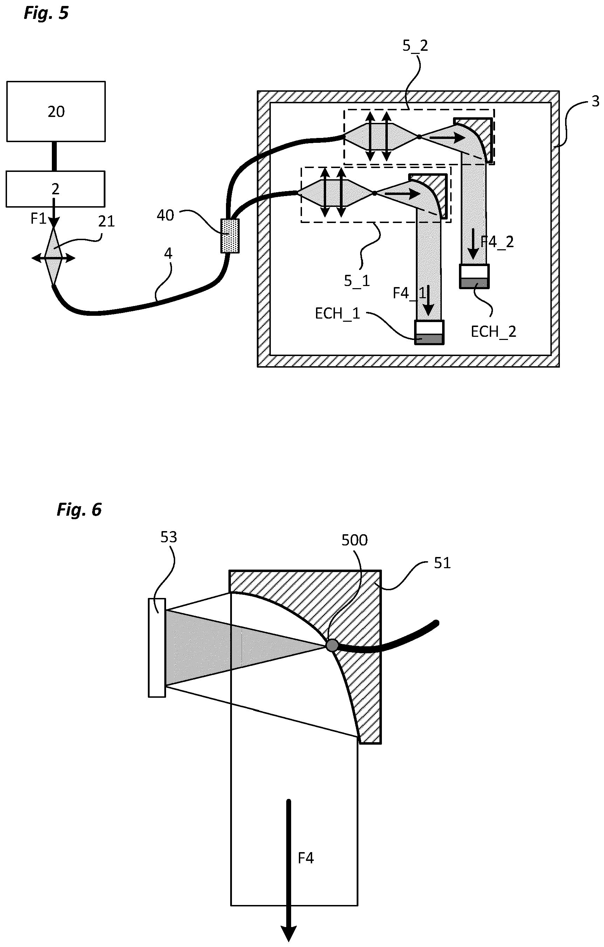

Une variante de réalisation avantageuse illustrée par la figure 5 permet, en utilisant une seule source lumineuse 2, de venir éclairer plusieurs contenants 1. Pour cela, le système intègre un séparateur 40 (« splitter » en anglais) agencé sur le guide optique 4 et ayant pour fonction de diviser le faisceau lumineux d'entrée F1 émis par la source lumineuse 2 en entrée du guide optique 4 en plusieurs faisceaux lumineux de sortie. Le système comporte alors plusieurs dispositifs optiques 5_1, 5_2 en parallèle, chargés chacun de générer un faisceau lumineux parallèle de stimulation F4_1, F4_2 distinct à destination d'un contenant 1 particulier. On vient ainsi stimuler optiquement plusieurs échantillons biologiques ECH1, ECH2 en parallèle.

Une autre variante de réalisation représentée sur la figure 6 réside dans la réalisation du dispositif optique. Selon cette variante de réalisation, la source divergente 500 (créée par l'ensemble optique 50) est positionnée dans le miroir parabolique hors axe 51 (qui est alors percé) et on ajoute un miroir 53 de renvoi (miroir plan) qui réfléchit alors le faisceau divergent incident vers le miroir parabolique hors axe 51. Une fibre optique peut être employée pour véhiculer la lumière jusqu'au point formant la source divergente 500. Cette solution est plus complexe à mettre en place mais aussi plus compacte.

### Avantages

L'invention présente ainsi de nombreux avantages, parmi lesquels :
- Elle permet d'éclairer une zone d'intérêt de manière identique en tout point afin que chaque cellule biologique soit stimulée avec la même intensité et la même incidence de l'onde lumineuse ;
- Elle permet de résoudre les problèmes thermiques présents dans l'enceinte fermée, en déportant la source lumineuse et sa commande à l'extérieur ;
- Elle permet de créer un faisceau homogène qui coïncide parfaitement avec la zone à éclairer ;
- Elle permet de venir éclairer plusieurs échantillons biologiques en parallèle, avec des faisceaux lumineux identiques ;
- Elle est simple à mettre en oeuvre et peut offrir des possibilités de réglage tenant compte des dimensions des zones à éclairer ;

## Revendications

1. Système de stimulation lumineuse d'un échantillon biologique (ECH), comprenant :
- Une source lumineuse (2) apte à émettre un faisceau lumineux, dit faisceau lumineux d'entrée (F1),
- Une enceinte (3) fermée destinée à recevoir au moins un contenant (1) dans lequel est placé l'échantillon biologique,
- **Caractérisé en ce que** :
- La source lumineuse (2) est placée à l'extérieur de l'enceinte (3),
- Le système comporte un dispositif optique (5) placé à l'intérieur de l'enceinte (3),
- Le système comporte un guide optique (4) connecté d'un côté sur ladite source lumineuse (2) pour recevoir ledit faisceau lumineux d'entrée (F1) et de l'autre côté sur ledit dispositif optique (5), le guide optique (4) étant agencé pour guider le faisceau lumineux d'entrée émis par la source lumineuse jusqu'au dispositif optique (5) et émettre un faisceau lumineux, dit faisceau lumineux de sortie (F2), à destination du dispositif optique,
- Ledit dispositif optique étant configuré pour générer un faisceau lumineux parallèle de stimulation (F4) de l'échantillon biologique (ECH) avec une section constante déterminée, à partir dudit faisceau lumineux de sortie (F2).

2. Système selon la revendication 1, **caractérisé en ce que** le dispositif optique (5) comporte un ensemble optique (50) configuré pour générer un faisceau lumineux divergent (F3) à partir du faisceau lumineux de sortie (F2) et un miroir parabolique hors axe (51) positionné par rapport audit ensemble optique pour réfléchir ledit faisceau lumineux divergent et former ledit faisceau lumineux parallèle de stimulation (F4).

3. Système selon la revendication 2, **caractérisé en ce qu'**il comporte des moyens de réglage de la position dudit ensemble optique (50) par rapport à celle du miroir parabolique hors axe (51).

4. Système selon la revendication 2 ou 3, **caractérisé en ce qu'**il comporte des moyens de réglage de la position du miroir parabolique hors axe (51) par rapport à celle dudit ensemble optique (50).

5. Système selon l'une des revendications 2 à 4, **caractérisé en ce qu'**il comporte des moyens de réglage de l'orientation dudit ensemble optique (50) par rapport au miroir parabolique hors axe (51).

6. Système selon l'une des revendications 1 à 5, **caractérisé en ce qu'**il comporte des moyens de réglage de l'orientation du miroir parabolique hors axe (51) par rapport audit ensemble optique (50).

7. Système selon l'une des revendications 1 à 6, **caractérisé en ce que** la source lumineuse (2) est une source laser régulée.

8. Système selon l'une des revendications 1 à 7, **caractérisé en ce qu'**il comporte un masque agencé en sortie du dispositif optique (5) pour mettre en forme la section du faisceau lumineux parallèle de stimulation (F4).

9. Système selon l'une des revendications 1 à 8, **caractérisé en ce que** le guide d'onde (4) comporte un séparateur (40), agencé pour séparer ledit faisceau lumineux d'entrée (F1) en ledit faisceau lumineux de sortie et un deuxième faisceau lumineux de sortie, et **en ce que** le système comporte un deuxième dispositif optique (5_2) de mise en forme du deuxième faisceau lumineux de sortie, configuré pour générer un deuxième faisceau lumineux de stimulation (F4_2).

10. Système selon l'une des revendications 1 à 9, **caractérisé en ce que** l'enceinte (3) fermée est un incubateur.

11. Procédé de stimulation lumineuse d'un échantillon biologique, mis en oeuvre à l'aide du système tel que défini dans l'une des revendications 1 à 10, **caractérisé en ce qu'**il comporte les étapes suivantes :
- Positionner dans ladite enceinte (3) fermée un contenant (1) dans lequel est placé ledit échantillon biologique (ECH) à stimuler, ledit contenant (1) comportant une fenêtre d'illumination (10) ayant une section d'illumination par laquelle peut pénétrer un faisceau lumineux, ce faisceau lumineux étant un faisceau lumineux parallèle de stimulation (F4) de l'échantillon biologique,
- Configurer le dispositif optique (5) pour obtenir le faisceau lumineux parallèle de stimulation (F4) avec une section constante de surface supérieure ou égale à la section d'illumination (10) de la fenêtre d'illumination du contenant,
- Activer ladite source lumineuse (2) pour émettre le faisceau lumineux d'entrée à l'intérieur du guide optique (4) et obtenir le faisceau lumineux de sortie à appliquer au dispositif optique pour la mise en forme de ce faisceau.

12. Procédé selon la revendication 11, **caractérisé en ce que** le contenant (1) est une boîte de Pétri.
